(19) Europäisches Patentamt
European Patent Office
Office européen des brevets

(11) EP 1 366 111 B2

(12) # NEUE EUROPÄISCHE PATENTSCHRIFT
Nach dem Einspruchsverfahren

(45) Veröffentlichungstag und Bekanntmachung des Hinweises auf die Entscheidung über den Einspruch:
**29.04.2020 Patentblatt 2020/18**

(45) Hinweis auf die Patenterteilung:
**04.04.2012 Patentblatt 2012/14**

(21) Anmeldenummer: **02719706.0**

(22) Anmeldetag: **18.01.2002**

(51) Int Cl.:
***C08K 3/32*** (2006.01) ***A61L 15/60*** (2006.01)

(86) Internationale Anmeldenummer:
**PCT/EP2002/000484**

(87) Internationale Veröffentlichungsnummer:
**WO 2002/060983 (08.08.2002 Gazette 2002/32)**

(54) **WASSERABSORBIERENDES MITTEL, VERFAHREN ZU SEINER HERSTELLUNG UND SEINE VERWENDUNG**

WATER-ABSORBING AGENT, METHOD FOR THE PRODUCTION AND THE UTILIZATION THEREOF

PRODUIT HYDROPHILE, SON PROCEDE DE PRODUCTION ET SON UTILISATION

(84) Benannte Vertragsstaaten:
**AT BE CH CY DE DK ES FI FR GB GR IE IT LI LU MC NL PT SE TR**

(30) Priorität: **19.01.2001 DE 10102429**

(43) Veröffentlichungstag der Anmeldung:
**03.12.2003 Patentblatt 2003/49**

(73) Patentinhaber: **BASF SE**
**67056 Ludwigshafen am Rhein (DE)**

(72) Erfinder:
- **DANIEL, Thomas**
  **67165 Waldsee (DE)**
- **RIEGEL, Ulrich**
  **60386 Frankfurt (DE)**
- **WEISMANTEL, Matthias**
  **63637 Jossgrund (DE)**

(74) Vertreter: **BASF IP Association**
**BASF SE**
**GBI-C006**
**67056 Ludwigshafen (DE)**

(56) Entgegenhaltungen:
**EP-A1- 0 441 975** **EP-A1- 0 605 215**
**WO-A-92/00108** **WO-A-94/15651**
**JP-A- S61 125 436**

- **DATABASE WPI Section Ch, Week 199534 Derwent Publications Ltd., London, GB; Class A60, AN 1995-261412 XP002207495 & JP 07 165981 A (SEKISUI PLASTICS CO LTD) , 27. Juni 1995 (1995-06-27)**

EP 1 366 111 B2

## Beschreibung

Die vorliegende Erfindung betrifft ein wasserabsorbierendes Mittel, das Teilchen eines wasserabsorbierenden Polymerisats umfasst, ein Verfahren zu seiner Herstellung und die Verwendung des wasserabsorbierenden Mittels zur Absorption von Körperflüssigkeiten, zur Herstellung von Hygieneartikeln und zur Bodenverbesserung.

Wasserabsorbierende Polymerisate, die auch als Hydrogel-bildende Polymerisate oder Superabsorber (Superabsorbing Polymers, im Folgenden als SAP abgekürzt) bezeichnet werden, sind bekannt. Hierbei handelt es sich um Netzwerke flexibler hydrophiler Polymerisate, die sowohl ionischer als auch nichtionischer Natur sein können. Diese sind in der Lage, wässrige Flüssigkeiten unter Bildung eines Hydrogels zu absorbieren und damit zu binden. Einen umfassenden Überblick über SAP, ihre Anwendung und ihre Herstellung gibt F.L. Buchholz und A.T. Graham (Herausgeber) in "Modern Superabsorbent Polymer Technology", Wiley-VCH, New York, 1998.

SAP finden insbesondere in Hygieneartikeln wie Windeln, Inkonti-nenzeinlagen und -hosen, Damenbinden und dergleichen Verwendung zur Absorption von Körperflüssigkeiten. Problematisch ist hierbei vielfach, dass der Superabsorber an der Eintrittsstelle der Flüssigkeit stark quillt und eine Sperrschicht für nachfolgende Flüssigkeitsmengen bildet. Hierdurch wird eine Weiterleitung und Verteilung der Flüssigkeit im Absorptionskern verhindert. Diese Eigenart des Superabsorbers wird auch als "Gelblocking-Effekt" bezeichnet. Nachfolgende Flüssigkeitsmengen werden dann nicht mehr vom Absorptionskern aufgenommen, und es kommt zur unkontrollierten Verteilung der Flüssigkeit auf der Windeloberfläche und im Extremfall zum Austritt der Flüssigkeit.

Die DE-A-3 523 617, US-A-4,734,478 und US-4,286,082 beschreiben wasserabsorbierende Harze, die Siliciumdioxid beigemengt enthalten. Das zugesetzte Siliciumdioxid soll die Verbackungsneigung des Harzes herabsetzen. In der WO 87/00848 ist die Mitverwendung kolloidaler Trägermaterialien, wie kolloidale Kieselsäure, zur Erhöhung der Gelstärke beschrieben.

Die WO 95/11932 offenbart die Zugabe feinteiliger Kieselsäure zur Oberflächennachvernetzerlösung. Die EP-386 897 offenbart die Verwendung polyvalenter Metallionen als Vernetzer. Die polyvalenten Metalle werden in Form ihrer wasserlöslichen Salze eingesetzt. Das Eigenschaftsprofil der beschriebenen wasserabsorbierenden Mittel ist jedoch nicht in allen Punkten zufriedenstellend.

Der vorliegenden Erfindung liegt daher die Aufgabe zugrunde, wasserabsorbierende Mittel bereitzustellen, die sich durch verbesserte Anwendungseigenschaften, insbesondere durch hohe Absorptionskapazitäten unter Druck, verbesserte Flüssigkeitstransportleistung sowie schnellere Anquellgeschwindigkeit, auszeichnen.

Erfindungsgemäß wird diese Aufgabe durch ein wasserabsorbierendes Mittel gelöst, das Teilchen eines oberflächennachvernetzten wasserabsorbierenden Polymerisats umfasst, deren Oberfläche mit einem wasserunlöslichen Metallphosphat einer mittleren Teilchengröße von 2 bis weniger als 400 $\mu$m assoziiert ist.

Die Erfindung betrifft außerdem ein Verfahren zur Herstellung des wasserabsorbierenden Mittels, wobei man

a) ein teilchenförmiges wasserabsorbierendes Polymerisat mit einem feinteiligen wasserunlöslichen Metallphosphat innig vermischt; oder

b) eine Aufschlämmung eines feinteiligen wasserunlöslichen Metallphosphats auf ein teilchenförmiges wasserabsorbierendes Polymerisat aufbringt; oder

c) eine Phosphationen enthaltende erste wässrige Lösung mit einer zweiten wässrigen Lösung in Gegenwart eines teilchenförmigen wasserabsorbierenden Polymerisats in Kontakt bringt, wobei die zweite Lösung ein wasserlösliches Salz eines ein wasserunlösliches Phosphat bildenden Metalls enthält.

Zweckmäßigerweise weist das erfindungsgemäße wasserabsorbierende Mittel im Allgemeinen eine Saline Flow Conductivity (SFC) von wenigstens 30 x $10^{-7}$ $cm^3 \cdot s/g$, eine Absorption unter Druck (AUL) (4,8 kPa; 0,7 psi) von wenigstens 20 g/g sowie eine Zentrifugenretensionskapazität (CRC) von wenigstens 24 g/g auf.

Der Begriff "assoziiert" ist im weitesten Sinn zu verstehen und soll jede Art der Wechselwirkung zwischen dem wasserunlöslichen

Metallphosphat und der Oberfläche der Teilchen des Polymerisats umfassen. Man versteht hierunter, dass das wasserunlösliche Metallphosphat nahezu ausschließlich auf der Oberfläche der Teilchen des wasserabsorbierenden Polymerisats angeordnet ist und nicht oder nur in unwesentlicher Menge im Volumen der Teilchen. In der Regel ist das wasserunlösliche Metallphosphat durch physikalische Wechselwirkung assoziert, z. B. in Oberflächenunebenheiten oder oberflächennahe Hohlräume eingeschlossen.

Zu den geeigneten wasserunlöslichen Metallphosphaten zählen auch solche Phosphate, die im technischen Sinn als "Phosphate" angesehen werden können und z. B. als gemischte Phosphat-Oxide, Phosphat-Hydroxide, Phosphat-Silicate, Phosphat-Fluoride oder dergleichen aufgefasst werden können.

Bevorzugte wasserunlösliche Metallphosphate sind solche, die ein Phosphat der Formel $M_4P_2O_7$, $M_2HPO_4$

oder $M_3PO_4$ umfassen, worin M für ein Äquivalent eines unter Calcium, Magnesium, Strontium, Barium, Zink, Eisen, Aluminium, Titan, Zirkonium, Hafnium, Zinn, Cer, Scandium, Yttrium oder Lanthan oder Gemischen davon ausgewählten Metalls steht. M kann auch Alkalimetallphosphate umfassen, solange das gemischte Phosphat wasserunlöslich ist.

Bevorzugte Phosphate sind Calciumhydrogenphosphat, tertiäres Calciumphosphat, Apatit, Thomasmehl der Formel $Ca_5(PO_4)[SiO_4]$, Berlinit der Formel $AlPO_4$, Rhenaniaphosphat der Formel $3CaNaPO_4 \cdot Ca_2SiO_4$. Besonders bevorzugt sind tertiäres Calciumphosphat, Calciumhydrogenphosphat sowie Apatit. Unter dem Begriff Apatit versteht man die Fluor-, Hydroxyl-, Chlor-, Carbonat- und Carbonat-Fluorapatite. Es können selbstverständlich auch Gemische verschiedener wasserunlöslicher Metallphosphate eingesetzt werden.

Ist die Verwendung des wasserabsorbierenden Mittels in einem Hygieneartikel angestrebt, kommen insbesondere physiologisch unbedenkliche wasserunlösliche Metallphosphate und deren Gemische in Betracht, wie tertiäres Calciumphosphat, Hydroxylapatit oder Calciumhydrogenphosphat.

Üblicherweise beträgt der Anteil des wasserunlöslichen Metallphosphats etwa 0,001 bis 10 Gew.-%, vorzugsweise 0,01 bis 5 Gew.-% und ganz besonders bevorzugt 0,05 bis 2,5 Gew.-%, bezogen auf das Gewicht des wasserabsorbierenden Polymerisats.

Geeignete wasserabsorbierende Polymerisate sind insbesondere Polymerisate hydrophiler Monomere, Pfropf (co)polymere eines oder mehrerer hydrophiler Monomere auf einer geeigneten Pfropfgrundlage, vernetzte Cellulose- oder Stärkeether, vernetzte Carboxymethylcellulose, teilweise vernetzte Polyether oder in wässrigen Flüssigkeiten quellbare Naturprodukte, wie Guarderivate, Alginate oder Carrageenane.

Geeignete Pfropfgrundlagen können natürlichen oder synthetischen Ursprungs sein. Hierzu zählen Stärken, d. h. native Stärken aus der Gruppe der Maisstärke, Kartoffelstärke, Weizenstärke, Reisstärke, Tapiokastärke, Sorghunstärke, Maniokstärke, Erbsenstärke oder deren Mischungen, modifizierte Stärken, Stärkeabbauprodukte, z. B. oxidativ, enzymatisch oder hydrolytisch abgebaute Stärken, Dextrine, z. B. Röstdextrine sowie niedere Oligo- und Polysaccharide, z. B. Cyclodextrine mit 4 bis 8 Ringgliedern. Als Oligo-und Polysaccharide kommen weiterhin Cellulose, Stärke- und Cellulosederivate in Betracht. Ferner eignen sich Polyvinylalkohole, Polyamine, Polyamide, hydrophile Polyester oder Polyalkylenoxide, insbesondere Polyethylenoxid und Polypropylenoxid. Geeignete Polyalkylenoxide weisen die allgemeine Formel I auf,

$$R^1 - O - (CH_2 - \underset{}{\overset{X}{\overset{|}{C}}}H - O)_n - R^2 \qquad (I)$$

worin

- $R^1$, $R^2$ unabhängig voneinander für Wasserstoff; $C_1$-$C_4$-Alkyl; $C_2$-$C_6$-Alkenyl; Aryl, insbesondere Phenyl; oder (Meth) acryloyl stehen;
- X für Wasserstoff oder Methyl und
- n für eine ganze Zahl von 1 bis 1000, insbesondere 10 bis 400 steht.

Vorzugsweise verwendet man als wasserabsorbierende Polymerisate Polymere monoethylenisch ungesättigter Säuren. Diese liegen vorzugsweise zumindest teilweise in Form ihrer Salze, insbesondere der Alkalimetallsalze, wie Natrium- oder Kaliumsalze, oder als Ammoniumsalze vor. Derartige Polymerisate quellen beim Kontakt mit wässrigen Flüssigkeiten besonders gut zu Gelen auf.

Besonders bevorzugt sind vernetzte wasserabsorbierende Polymerisate monoethylenisch ungesättigter $C_3$-$C_6$-Carbonsäuren und/oder deren Alkali- oder Ammoniumsalze. Insbesondere werden vernetzte Polyacrylsäuren bevorzugt, deren Säurengruppen zu 25 bis 100 % als Alkali- oder Ammoniumsalze vorliegen.

Derartige Polymere erhält man z. B. durch Polymerisation monoethylenisch ungesättigter Säuren oder deren Salzen in Gegenwart von Vernetzern. Allerdings kann man auch ohne Vernetzer polymerisieren und nachträglich vernetzen.

Das wasserabsorbierende Polymerisat ist vorzugsweise aufgebaut aus

- 49,9 bis 99,9 Gew.-% wenigstens eines unter monoethylenisch ungesättigten Säuren und deren Salzen ausgewählten Monomers A,

- 0 bis 50 Gew.-%, vorzugsweise 0 bis 20 Gew.-% wenigstens eines von dem Monomeren A verschiedenen, nicht vernetzend wirkenden monoethylenisch ungesättigten Monomers B und

- 0,001 bis 20 Gew.-%, vorzugsweise 0,01 bis 14 Gew.-% wenigstens eines vernetzend wirkenden Monomers C.

Zu den Monomeren A zählen monoethylenisch ungesättigte Mono- und Dicarbonsäuren mit 3 bis 25 vorzugsweise 3 bis 6 C-Atomen, die auch in Form ihrer Salze oder Anhydride eingesetzt werden können. Beispiele hierfür sind Acrylsäure, Methacrylsäure, Ethacrylsäure, $\alpha$-Chloracrylsäure, Crotonsäure, Maleinsäure, Maleinsäureanhydrid, Itaconsäure, Citraconsäure, Mesaconsäure, Glutaconsäure, Aconitsäure und Fumarsäure. Zu den Monomeren A zählen weiterhin die Halbester von monoethylenisch ungesättigten Dicarbonsäuren mit 4 bis 10 vorzugsweise 4 bis 6 C-Atomen, z. B. von Maleinsäure wie Maleinsäuremonomethylester. Zu den Monomeren A zählen auch monoethylenisch ungesättigte Sulfonsäuren und Phosphonsäuren, beispielsweise Vinylsulfonsäure, Allylsulfonsäure, Sulfoethylacrylat, Sulfoethylmethacrylat, Sulfopropylacrylat, Sulfopropylmethacrylat, 2-Hydroxy-3-acryloxypropylsulfonsäure, 2-Hydroxy-3-methacryloxypropylsulfonsäure, Styrolsulfonsäure, 2-Acrylamido-2-methylpropansulfonsäure, Vinylphosphonsäure und Allylphosphonsäure und die Salze, insbesondere die Natrium-, Kalium- und Ammoniumsalze dieser Säuren. Die Monomere A können als solche oder als Mischungen untereinander eingesetzt werden. Die angegebenen Gewichtsanteile beziehen sich sämtlich auf die Säureform.

Bevorzugte Monomere A sind Acrylsäure, Methacrylsäure, Vinylsulfonsäure, 2-Acrylamido-2-methylpropansulfonsäure oder Mischungen dieser Säuren. Bevorzugte Monomere A sind Acrylsäure und Mischungen der Acrylsäure mit anderen Monomeren A, z. B. Mischungen aus Acrylsäure und Methacrylsäure, aus Acrylsäure und Acrylamidopropansulfonsäure oder aus Acrylsäure und Vinylsulfonsäure. Besonders bevorzugt umfassen die Monomere A als Hauptbestandteil Acrylsäure.

Zur Optimierung von Eigenschaften der erfindungsgemäßen Polymerisate kann es sinnvoll sein, zusätzliche monoethylenisch ungesättigte Monomere B einzusetzen, die von den Monomeren A verschieden sind, d. h. die keine Säuregruppen tragen, aber mit den Monomeren A copolymerisierbar sind und nicht vernetzend wirken. Hierzu gehören beispielsweise monoethylenisch ungesättigte Nitrile wie Acrylnitril, Methacrylnitril, die Amide der vorgenannten monoethylenisch ungesättigten Carbonsäuren, z. B. Acrylamid, Methacrylamid, N-Vinylamide wie N-Vinylformamid, N-Vinylacetamid, N-Methyl-vinylacetamid, N-Vinylpyrrolidon und N-Vinylcaprolactam. Zu den Monomeren zählen außerdem Vinylester gesättigter $C_1$-$C_4$-Carbonsäuren wie Vinylformiat, Vinylacetat und Vinylpropionat, Alkylvinylether mit mindestens 2 C-Atomen in der Alkylgruppe, z. B. Ethylvinylether oder Butylvinylether, Ester monoethylenisch ungesättigter $C_3$-$C_6$-Carbonsäuren, z. B. Ester aus einwertigen $C_1$-$C_{18}$-Alkoholen und Acrylsäure, Methacrylsäure oder Maleinsäure, Acrylsäure- und Methacrylsäureester von alkoxylierten einwertigen, gesättigten Alkoholen, z. B. von Alkoholen mit 10 bis 25 C-Atomen, die mit 2 bis 200 Mol Ethylenoxid und/oder Propylenoxid pro Mol Alkohol umgesetzt worden sind, sowie Monoacrylsäureester und Monomethacrylsäureester von Polyethylenglykol oder Polypropylenglykol, wobei die Molmassen ($M_n$) der Polyalkylenglykole beispielsweise bis zu 2000 betragen können. Weiterhin geeignete Monomere sind Styrol und alkylsubstituierte Styrole wie Ethylstyrol oder tert-Butylstyrol.

Die Monomere B können auch als Mischungen untereinander eingesetzt werden, z. B. Mischungen aus Vinylacetat und 2-Hydroxyethylacrylat in beliebigem Verhältnis.

Als vernetzend wirkende Monomere C kommen solche Verbindungen in Betracht, die mindestens zwei, z. B. 2, 3, 4 oder 5 ethylenisch ungesättigte Doppelbindungen im Molekül aufweisen. Beispiele für Verbindungen dieses Typs sind N,N'-Methylenbisacrylamid, Polyethylenglykoldiacrylate und Polyethylenglykoldimethacrylate, die sich jeweils von Polyethylenglykolen eines Molekulargewichts von 106 bis 8500, vorzugsweise 400 bis 2000, ableiten, Trimethylolpropantriacrylat, Trimethylolpropantrimethacrylat, Ethylenglykoldiacrylat, Ethylenglykoldimethacrylat, Propylenglykoldiacrylat, Propylenglykoldimethacrylat, Butandioldiacrylat, Butandioldimethacrylat, Hexandioldiacrylat, Hexandioldimethacrylat, Diethylenglykoldiacrylat, Diethylenglykoldimethacrylat, Triethylenglykoldiacrylat, Triethylenglykoldimethacrylat, Dipropylenglykoldiacrylat, Dipropylenglykoldimethacrylat, Tripropylenglykoldiacrylat, Tripropylenglykoldimethacrylat, Allylmethacrylat, Diacrylate und Dimethacrylate von Blockcopolymerisaten aus Ethylenoxid und Propylenoxid, zwei-, drei-, vier- oder fünffach mit Acrylsäure oder Methacrylsäure veresterte mehrwertige Alkohole, wie Glycerin, Trimethylolpropan, Pentaerythrit oder Dipentaerythrit, Ester monoethylenisch ungesättigter Carbonsäuren mit ethylenisch ungesättigten Alkoholen wie Allylalkohol, Cyclohexenol und Dicyclopentenylalkohol, z.B. Allylacrylat und Allylmethacrylat, weiterhin Triallylamin, Dialkyldiallylammoniumhalogenide wie Dimethyldiallylammoniumchlorid und Diethyldiallylammoniumchlorid, Tetraallylethylendiamin, Divinylbenzol, Diallylphthalat, Polyethylenglykoldivinylether von Polyethylenglykolen eines Molekulargewichtes von 106 bis 4000, Trimethylolpropandiallylether, Butandioldivinylether, Pentaerythrittriallylether, Umsetzungsprodukte von 1 Mol Ethylenglykoldiglycidylether oder Polyethylenglykoldiglycidylether mit 2 Mol Pentaerythritoltriallylether oder Allylalkohol, und Divinylethylenharnstoff.

Davon sind wasserlösliche Monomere bevorzugt, d. h. Verbindungen, deren Wasserlöslichkeit bei 20 °C wenigstens 50 g/L beträgt. Hierzu zählen z. B. Polyethylenglykoldiacrylate und Polyethylenglykoldimethacrylate, Vinylether von Additionsprodukten von 2 bis 400 Mol Ethylenoxid an 1 Mol eines Diols oder Polyols, Ethylenglykoldiacrylat, Ethylenglykoldimethacrylat oder Triacrylate und Trimethacrylate von Additionsprodukten von 6 bis 20 Mol Ethylenoxid an 1 Mol Glycerin, Pentaerythrittriallylether und Divinylharnstoff.

Als Monomere C kommen weiterhin solche in Betracht, die wenigstens eine ethylenisch ungesättigte Doppelbindung sowie wenigstens eine weitere funktionelle Gruppe aufweisen, die hinsichtlich ihrer Reaktivität gegenüber Carboxylgruppen komplementär ist. Zu funktionellen Gruppen mit komplementärer Reaktivität gegenüber Carboxylgruppen zählen beispielsweise Hydroxyl-, Amino-, Epoxy- und Aziridinogruppen. Verwendung finden z. B. die Hydroxyalkylester der vorstehend erwähnten monoethylenisch ungesättigten Carbonsäuren, wie 2-Hydroxyethylacrylat, 3-Hydroxypropylacrylat, 4-Hydroxybutylacrylat, 2-Hydroxyethyl(meth)acrylat, 3-Hydroxypropyl(meth)acrylat und 4-Hydroxybutyl(meth)acrylat, Allylpiperidiniumbromid, N-Vinylimidazole, wie N-Vinylimidazol, 1-Vinyl-2-methylimidazol und N-Vinylimidazoline, wie N-Vinylimidazolin, 1-Vinyl-2-methylimidazolin, 1-Vinyl-2-ethylimidazolin oder 1-Vinyl-2-propylimidazolin, die in Form der freien Basen, in quaternisierter Form oder als Salz bei der Polymerisation verwendet werden. Außerdem eignen sich Dimethylaminoethylacrylat, Dimethylaminoethylmethacrylat, Diethylaminoethylacrylat oder Diethylaminoethylmethacrylat. Diese basischen Ester werden vorzugsweise in quarternisierter Form oder als Salz eingesetzt. Weiterhin eignet sich auch Glycid(meth)acrylat.

Als vernetzende Monomere C können ferner Verbindungen fungieren, die wenigstens zwei funktionelle Gruppen aufweisen, die hinsichtlich ihrer Reaktivität gegenüber der Carboxylgruppe des Polymers komplementär sind. Geeignete funktionelle Gruppen sind die bereits vorstehend genannten funktionellen Gruppen, wie Hydroxyl-, Amino-, Epoxy und Aziridingruppen sowie Isocyanat-, Ester- und Amidogruppen. Zu den geeigneten Vernetzern dieses Typs zählen beispielsweise Aminoalkohole, wie Ethanolamin oder Triethanolamin, Di- und Polyole, wie 1,3-Butandiol, 1,4-Butandiol, Ethylenglykol, Diethylenglykol, Triethylenglykol, Tetraethylenglykol, Polyethylenglykol, Glycerin, Polyglycerin, Propylenglykol, Polypropylenglykol, Trimethylolpropan, Pentaerythrit, Polyvinylalkohol, Sorbit, Stärke, Blockcopolymerisate aus Ethylenoxid und Propylenoxid, Polyamine wie Ethylendiamin, Diethylentriamin, Triethylentetramin, Tetraethylenpentamin, Pentaethylenhexamin und Polyethylenimine sowie Polyamine mit Molmassen von jeweils bis zu 4000000, Ester wie Sorbitanfettsäureester, ethoxylierte Sorbitanfettsäureester, Polyglycidylether wie Ethylenglykoldiglycidylether, Polyethylenglykol-diglycidylether, Glycerindiglycidylether, Glycerinpolyglycidylether, Diglycerinpolyglycidylether, Polyglycerinpolyglycidylether, Sorbitpolyglycidylether, Pentaerythritpolyglycidylether, Propylenglykoldiglycidylether und Polypropylenglykoldiglycidylether, Polyaziridinverbindungen wie 2,2-Bishydroxymethylbutanol-tris[3-(1-aziridinyl)-propionat], Diamide der Kohlensäure, wie 1,6-Hexamethylendiethylenharnstoff, Diphenylmethan-bis-4,4'-N,N'-diethylenharnstoff, Halogenepoxyverbindungen, wie Epichlorhydrin und $\alpha$-Methylepifluorhydrin, Polyisocyanate, wie 2,4-Toluylendiisocyanat und Hexamethylendiisocyanat, Alkylencarbonate wie 1,3-Dioxolan-2-on und 4-Methyl-1,3-dioxolan-2-on, weiterhin Bisoxazoline und Oxazolidone, Polyamidoamine sowie deren Umsetzungsprodukte mit Epichlorhydrin, ferner polyquaternäre Amine, wie Kondensationsprodukte von Dimethylamin mit Epichlorhydrin, Homo- und Copolymere von Diallyldimethylammoniumchlorid sowie Homo- und Copolymerisate von Dimethylaminoethyl(meth)acrylat, die gegebenenfalls mit beispielsweise Methylchlorid quaterniert sind.

Die wasserabsorbierenden Polymerisate können hergestellt werden, indem man die Monomere A, B und C, gegebenenfalls in Gegenwart einer geeigneten Pfropfgrundlage, in wässriger Lösung einer radikalischen Polymerisation unterwirft. Die Polymerisation kann sowohl in homogener wässriger Phase als auch als Suspensionspolymerisation erfolgen, wobei die wässrige Lösung der Monomere die disperse Phase bildet.

Bevorzugt ist die Polymerisation in wässriger Lösung als sogenannte Gelpolymerisation. Hierzu wird z. B. eine 10 bis 70 gew.-%ige wässrige Lösung der Monomere A, B und C, gegebenenfalls in Gegenwart einer geeigneten Pfropfgrundlage, mittels eines Polymerisationsinitiators unter Ausnutzung des Trommsdorff-Norrish-Effektes polymerisiert.

Die Polymerisation erfolgt in der Regel im Temperaturbereich von 0 °C und 150 °C, vorzugsweise im Bereich von 10 °C und 100 °C, und kann sowohl bei Normaldruck als auch unter erhöhtem oder erniedrigtem Druck durchgeführt werden. Wie üblich kann die Polymerisation auch in einer Schutzgasatmosphäre, vorzugsweise unter Stickstoff, ausgeführt werden.

Als technische Verfahren zur Herstellung dieser Produkte können alle Verfahren Anwendung finden, die üblicherweise bei der Herstellung von Superabsorbern eingesetzt werden. Geeignete Maßnahmen sind z. B. in "Modern Superabsorbent Polymer Technology", F.L. Buchholz and A.T. Graham, Wiley-VCH, 1998, Kapitel 3, erläutert, worauf hiermit Bezug genommen wird. Als Polymerisationsreaktoren kommen die zur Herstellung üblichen Reaktoren, im Falle der Lösungspolymerisation insbesondere Bandreaktoren und Kneter, in Betracht (siehe "Modern Superabsorbent Polymer Technology", Kapitel 3.2.3). Die Polymerisate werden besonders bevorzugt nach einem kontinuierlichen oder diskontinuierlichen Knetverfahren hergestellt.

Als Initiatoren kommen grundsätzlich alle Verbindungen in Betracht, die beim Erwärmen auf Polymerisationstemperartur unter Bildung von Radikalen zerfallen. Die Polymerisation kann auch durch Einwirkung energiereicher Strahlung, z. B. UV-Strahlung, in Gegenwart von Photoinitiatoren ausgelöst werden. Auch eine Initiierung der Polymerisation durch Einwirkung von Elektronenstrahlen auf die polymerisierbare, wässrige Mischung ist möglich.

Geeignete Initiatoren sind beispielsweise Peroxoverbindungen wie organische Peroxide, organische Hydroperoxide, Wasserstoffperoxid, Persulfate, Perborate, Azoverbindungen und die sogenannten Redoxkatalysatoren. Bevorzugt werden wasserlösliche Initiatoren. In manchen Fällen ist es vorteilhaft, Mischungen verschiedener Polymerisationsinitiatoren zu verwenden, z. B. Mischungen aus Wasserstoffperoxid und Natrium- oder Kaliumperoxodisulfat. Geeignete organische Peroxide sind beispielsweise Acetylacetonperoxid, Methylethylketonperoxid, tert-Butylhydroperoxid, Cumolhydroperoxid, tert-Amylperpivalat, tert-Butylperpivalat, tert-Butylperneohexanoat, tert-Butylperisobutyrat, tert-Butylper-2-ethylhexanoat, tert-Butylperisononanoat, tert-Butylpermaleat, tert-Butylperbenzoat, Di-(2-ethylhexyl)peroxidicarbonat, Dicyclohexylperoxidicarbonat, Di-(4-tert.-butylcyclohexyl)peroxidicarbonat, Dimyristilperoxidicarbonat, Diacetylperoxi-dicarbonat, Allylperester, Cumylperoxyneodecanoat, tert.-Butylper-3,5,5-trimethylhexanoat, Acetylcyclohexylsulfonylperoxid, Dilaurylperoxid, Dibenzoylperoxid und tert.-Amylperneodekanoat. Besonders geeignete Polymerisationsinitiatoren sind wasserlösliche Azostarter, z. B. 2,2'-Azo-bis-(2-amidinopropan)dihydrochlorid, 2,2'-Azobis-(N,N'-dimethylen)isobutyramidin-dihydrochlorid, 2-(Carbamoylazo)isobutyronitril, 2,2'-Azobis[2-(2'-imidazolin-2-yl)propan]dihydrochlorid und 4,4'-Azobis-(4-cyanovaleriansäure). Die genannten Polymerisationsinitiatoren werden in üblichen Mengen eingesetzt, z. B. in Mengen von 0,01 bis 5, vorzugsweise 0,05 bis 2,0 Gew.-%, bezogen auf die zu polymerisierenden Monomeren.

Die bevorzugten Redoxinitiatoren zählen zu den wasserlöslichen Initiatoren und enthalten als oxidierende Komponente mindestens eine der oben angegebenen Peroxoverbindungen und als reduzierende Komponente beispielsweise Ascorbinsäure, Glukose, Sorbose, Ammonium- oder Alkalimetallhydrogensulfit, -sulfit, -thiosulfat, -hyposulfit, -pyro-sulfit oder -sulfid, Metallsalze, wie Eisen(II)-ionen oder Natriumhydroxymethylsulfoxylat. Vorzugsweise verwendet man als reduzierende Komponente des Redoxkatalysators Ascorbinsäure oder Natriumsulfit. Bezogen auf die bei der Polymerisation eingesetzte Menge an Monomeren verwendet man beispielsweise 3 x $10^{-6}$ bis 1 Mol-% der reduzierenden Komponente des Redoxkatalysatorsystems und 0,001 bis 5,0 Mol-% der oxidierenden Komponente des Redoxkatalysators.

Wenn man die Polymerisation durch Einwirkung energiereicher Strahlung auslöst, verwendet man üblicherweise als Initiator sogenannte Photoinitiatoren.

Bei der nachträglichen Vernetzung werden Polymere, die durch die Polymerisation der oben genannten monoethylenisch ungesättigten Säuren und gegebenfalls monoethylenisch ungesättigten Comonomere hergestellt wurden und die typischerweise ein Molekulargewicht größer 5000, bevorzugt größer 50000 aufweisen, mit Verbindungen umgesetzt, die mindestens zwei gegenüber Säuregruppen reaktive Gruppen aufweisen. Diese Umsetzung kann bei Raumtemperatur oder aber bei erhöhten Temperaturen bis zu 220 °C erfolgen. Als Vernetzer fungieren die vorstehend erwähnten Mononere C, die wenigstens zwei funktionelle Gruppe mit komplementärer Reaktivität gegenüber Carboxylgruppen aufweisen.

Zur nachträglichen Vernetzung werden die Vernetzer den erhaltenen Polymeren in Mengen von 0,5 bis 20 Gew.-%, bevorzugt von 1 bis 14 Gew.-%, bezogen auf die Menge des Polymers, zugesetzt.

Die erfindungsgemäßen Polymerisate fallen nach der Polymerisation in der Regel als Hydrogele mit einem Feuchtigkeitsgehalt von z. B. 0 bis 90 Gew.-%, meist 20 bis 90 Gew.-% an, die in der Regel zunächst nach bekannten Methoden grob zerkleinert werden. Die Grobzerkleinerung der Hydrogele erfolgt mittels üblicher Reißund/oder Schneidwerkzeuge, z. B. durch die Wirkung einer Austragspumpe im Falle der Polymerisation in einem zylindrischen Reaktor oder durch eine Schneidwalze oder Schneidwalzenkombination im Falle der Bandpolymerisation.

Sofern die Monomere A in nicht neutralisierter Form eingesetzt worden sind, kann man das erhaltene saure Polymerisat auf den gewünschten Neutralisationsgrad von in der Regel wenigstens 25 mol-%, vorzugsweise wenigstens 50 mol-%, vorzugsweise 50 bis 100 mol-%, bezogen auf Säuregruppen tragende Monomereinheiten, bringen. Alternativ kann die Einstellung des Neutralisationsgrades auch vor oder während der Polymerisation, z. B. im Kneter, vorgenommen werden.

Als Neutralisationsmittel kommen Alkalimetallbasen oder Ammoniak bzw. Amine in Frage. Vorzugsweise wird Natronlauge oder Kalilauge verwendet. Die Neutralisation kann jedoch auch mit Hilfe von Natriumcarbonat, Natriumhydrogencarbonat, Kaliumcarbonat oder Kaliumhydrogencarbonat oder anderen Carbonaten oder Hydrogencarbonaten oder Ammoniak vorgenommen werden. Darüberhinaus können für die Neutralisation primäre, sekundäre und tertiäre Amine eingesetzt werden.

Das so erhaltene, vorzugsweise (teil)neutralisierte Polymerisat wird anschließend bei erhöhter Temperatur, z. B. im Bereich von 80 °C bis 250 °C und insbesondere im Bereich von 100 °C bis 180 °C, nach bekannten Verfahren getrocknet (siehe "Modern Superabsorbent Polymer Technology" Kapitel 3.2.5). Hierbei erhält man die Polymerisate in Form von Pulvern oder Granulaten, die gegebenenfalls zur Einstellung der Partikelgrösse noch mehreren Mahl- und Siebvorgängen unterworfen werden (siehe "Modern Superabsorbent Polymer Technology" Kapitel 3.2.6 und 3.2.7).

Das erhaltene teilchenförmige Polymerisat wird anschließend oberflächennachvernetzt. Hierzu werden Verbindungen, die mit den sauren funktionellen Gruppen der Polymere unter Vernetzung reagieren können, vorzugsweise in Form einer wasserhaltigen Lösung auf die Oberfläche der Polymerisat-Partikel aufgebracht. Die wasserhaltige Lösung kann wassermischbare organische Lösungsmittel enthalten. Geeignete Lösungsmittel sind Alkohole wie Methanol, Ethanol, Isopropanol oder Aceton.

Geeignete Nachvernetzungsmittel sind beispielsweise:

- Di- oder Polyglycidylverbindungen wie Phosphonsäurediglycidylether oder Ethylenglykoldiglycidylether, Bischlorhydrinether von Polyalkylenglykolen,

- Alkoxysilylverbindungen,

- Polyaziridine, Aziridin-Einheiten enthaltende Verbindungen auf Basis von Polyethern oder substituierten Kohlenwasserstoffen, beispielsweise Bis-N-aziridinomethan,

- Polyamine oder Polyamidoamine sowie deren Umsetzungsprodukte mit Epichlorhydrin,

- Polyole wie Ethylenglykol, 1,2-Propandiol, 1,4-Butandiol, Glycerin, Methyltriglykol, Polyethylenglykole mit einem mittleren Molekulargewicht $M_w$ von 200 - 10000, Di- und Polyglycerin, Pentaerythrit, Sorbit, die Oxethylate dieser Polyole sowie deren Ester mit Carbonsäuren oder der Kohlensäure wie Ethylencarbonat oder Propylencarbonat,

- Kohlensäurederivate wie Harnstoff, Thioharnstoff, Guanidin, Dicyandiamid, 2-Oxazolidinon und dessen Derivate, Bisoxazolin, Polyoxazoline, Di- und Polyisocyanate,

- Di- und Poly-N-methylolverbindungen wie beispielsweise Methylenbis(N-methylol-methacrylamid) oder Melamin-FormaldehydHarze,

- Verbindungen mit zwei oder mehr blockierten Isocyanat-Gruppen wie beispielsweise Trimethylhexamethylendiisocyanat blockiert mit 2,2,3,6-Tetramethyl-piperidinon-4.

Bei Bedarf können saure Katalysatoren wie p-Toluolsulfonsäure, Phosphorsäure, Borsäure oder Ammoniumdihydrogenphosphat zugesetzt werden.

Besonders geeignete Nachvernetzungsmittel sind Di- oder Polyglycidylverbindungen wie Ethylenglykoldiglycidylether, die Umsetzungsprodukte von Polyamidoaminen mit Epichlorhydrin und 2-Oxazolidinon.

Das Aufbringen der Vernetzer-Lösung erfolgt bevorzugt durch Aufsprühen einer Lösung des Vernetzers in herkömmlichen Reaktionsmischern oder Misch- und Trocknungsanlagen wie beispielsweise Patterson-Kelly-Mischer, DRAIS-Turbulenzmischer, Lödige-Mischer, Schneckenmischer, Tellermischer, Wirbelschichtmischer und Schugi-Mix. Nach Aufsprühen der Vernetzer-Lösung kann ein Temperaturbehandlungsschritt nachfolgen, bevorzugt in einem nachgeschalteten Trockner, bei einer Temperatur zwischen 80 und 230°C, bevorzugt 80 bis 190°C, und besonders bevorzugt zwischen 100 und 160°C, über einen Zeitraum von 5 Minuten bis 6 Stunden, bevorzugt 10 Minuten bis 2 Stunden und besonders bevorzugt 10 Minuten bis 1 Stunde, wobei sowohl Spaltprodukte als auch Lösungsmittelanteile entfernt werden können. Die Trocknung kann aber auch im Mischer selbst erfolgen, durch Beheizung des Mantels oder Einblasen eines vorgewärmten Trägergases.

Zur Herstellung des erfindungsgemäßen wasserabsorbierenden Mittels geht man von einem teilchenförmigen wasserabsorbierenden Polymerisat aus, das in getrockneter Form oder als zerkleinertes Hydrogel mit dem oben angegebenen Feuchtigkeitsgehalt vorliegt und bringt auf der Oberfläche der Teilchen ein wasserunlösliches Metallphosphat auf.

Hierzu kann man das teilchenförmige wasserabsorbierende Polymerisat beispielsweise mit einem feinteiligen wasserunlöslichen Metallphosphat innig vermischen. Üblicherweise fügt man das feinteilige, wasserunlösliche Metallphosphat bei Raumtemperatur zum teilchenförmigen, wasserabsorbierenden Polymerisat und mischt solange, bis eine homogene Mischung vorliegt. Das Mischen kann unter Verwendung üblicher Vorrichtungen, beispielsweise mit einem Trommelmischer, Bandschneckenmischer oder Siloschneckenmischer, erfolgen. Das Vermischen mit dem feinteiligen wasserunlöslichen Metallphosphat kann vor oder nach einer Oberflächennachvernetzung erfolgen, z.B. während des Temperaturnachbehandlungsschrittes nach dem Aufbringen des Nachvernetzungsmittels.

Das feinteilige wasserunlösliche Metallphosphat weist eine mittlere Teilchengröße von 2 bis weniger als 400 $\mu$m, insbesondere von weniger als 100 $\mu$m, stärker bevorzugt weniger als 50 $\mu$m, besonders bevorzugt weniger als 10 $\mu$m und am stärksten bevorzugt im Bereich von 2 bis 7 $\mu$m auf.

Alternativ kann man eine Aufschlämmung eines feinteiligen wasserunlöslichen Metallphosphats auf ein teilchenförmiges wasserabsorbierendes Polymerisat aufbringen. Die Teilchengröße des wasserunlöslichen Metallphosphats ist vorzugsweise wie oben angegeben. Das Aufbringen der Aufschlämmung erfolgt beispielsweise durch Aufsprühen. Geeignete Dispersionsmittel zur Zubereitung der Aufschlämmung sind Wasser, organische Lösungsmittel, wie Alkohole, beispielsweise Methanol, Ethanol, Isopropanol, Ketone, beispielsweise Aceton, Methylethylketon oder Gemische von Wasser mit den vorstehend genannten organischen Lösungsmitteln. Das Aufbringen kann in herkömmlichen Reaktionsmischern oder Misch- und Trocknungsanlagen, wie sie vorstehend beschrieben sind, bei einer Temperatur zwischen Raumtemperatur und weniger als dem Siedepunkt des Dispersionsmittels, bevorzugt etwa Raumtemperatur, erfolgen. Zweckmäßigerweise kann man das Aufbringen der Aufschlämmung mit einer Oberfächennachvernetzung

kombinieren, indem man das feinteilige, wasserunlösliche Metallphosphat in der Lösung des Nachvernetzungsmittels dispergiert. Alternativ kann das Aufbringen der Aufschlämmung auch vor oder nach der Oberflächennachvernetzung erfolgen. An das Aufbringen der Aufschlämmung schließt sich gegebenenfalls ein Trocknungsschritt an.

In einer weiteren Ausführungsform der Erfindung erzeugt man in situ das wasserunlösliche Metallphosphat auf der Oberfläche des wasserabsorbierenden Polymerisats, indem man eine Phosphationen enthaltende erste wässrige Lösung mit einer zweiten wässrigen Lösung in Gegenwart eines teilchenförmigen wasserabsorbierenden Polymerisats in Kontakt bringt, wobei die zweite Lösung ein wasserlösliches Salz eines ein wasserunlösliches Phosphat bildenden Metalls enthält. Die Gegenionen für die Phosphationen enthaltende Lösung sind vorzugsweise Alkalimetallionen, wie Natrium- oder Kaliumionen. Bei der ersten Lösung kann es sich alternativ um verdünnte Phosphorsäure handeln. Die zweite Lösung enthält Ionen wenigstens eines Metallions, das ein wasserunlösliches Phosphat bildet, wie Calcium, Magnesium, Strontium, Barium, Zink, Eisen, Aluminium, Titan, Zirkonium, Hafnium, Zinn, Cer, Scandium, Yttrium oder Lanthan oder Gemische davon, vorzugsweise Calcium. Es liegt als Lösung eines ausreichend wasserlöslichen Salzes vor, z. B. als Halogenid, wie Chlorid, Bromid, Iodid, Hydroxid, Sulfat oder Nitrat. Das gewünschte wasserunlösliche Metallphosphat fällt beim Kontakt der ersten wässrigen Lösung mit der zweiten wässrigen Lösung durch Überschreiten seines Löslichkeitsproduktes aus. In einer besonders bevorzugten Ausführungsform verwendet man eine Lösung von Calciumchlorid als erste wässrige Lösung und eine Lösung von primärem, sekundärem oder tertiärem Natriumphosphat als zweite wässrige Lösung. Es entsteht in situ tertiäres Calciumphosphat, das sich langsam in Hydroxylapatit umwandelt. Die in situ-Ausfällung des wasserunlöslichen Metallphosphats auf der Oberfläche des wasserabsorbierenden Polymerisats kann vor, während oder nach der Oberflächennachbehandlung erfolgen. Gegebenenfalls schließt sich ein Trocknungsschritt an.

Die erfindungsgemäßen wasserabsorbierenden Mittel eignen sich hervorragend als Absorptionsmittel für Wasser und wässrige Flüssigkeiten, insbesondere Körperflüssigkeiten. Sie können mit Vorteil zur Herstellung von Hygieneartikeln wie Windeln, Inkonti-nenzeinlagen und -hosen, Tampons oder Damenbinden verwendet werden. Sie können ferner zur Bodenverbesserung, z.B. als Wasser zurückhaltendes Mittel im landwirtschaftlichen Gartenbau, verwendet werden.

Die folgenden Beispiele sollen die Erfindung näher erläutern, ohne sie jedoch einzuschränken.

I Beschreibung der Testmethoden

1. Zentrifugenretentionskapazität (CRC: Centrifuge Retention Capacity)

Hierbei wird die freie Quellbarkeit des Hydrogel-bildenden Polymerisats im Teebeutel bestimmt. Zur Bestimmung der CRC werden 0,2000 ± 0,0050 g getrocknetes Polymerisat (Kornfraktion 106 - 850 μm) in einem 60 x 85 mm großen Teebeutel eingewogen, der anschließend verschweißt wird. Der Teebeutel wird für 30 Minuten in 0,9 gew.%-ige Kochsalzlösung gegeben (mindestens 0,83 L Kochsalz-Lösung / 1 g Polymerpulver). Anschließend wird der Teebeutel 3 Minuten lang bei 250 G zentrifugiert und danach zur Bestimmung der absorbierten Flüssigkeitsmenge gewogen.

2. Absorption unter Druck (AUL Absorbency Under Load) (4,8 kPa; 0,7 psi)

Die Meßzelle zur Bestimmung der AUL 4,8 kPa (0,7 psi) ist ein Plexiglas-Zylinder mit einem Innendurchmesser von 60 mm und einer Höhe von 50 mm, der an der Unterseite einen angeklebten Edelstahl-Siebboden mit einer Maschenweite von 36 μm aufweist. Zu der Meßzelle gehört weiterhin eine Plastikplatte mit einem Durchmesser von 59 mm und ein Gewicht, welches zusammen mit der Plastikplatte in die Meßzelle hineingestellt werden kann. Das Gewicht der Plastikplatte und das Gewicht betragen zusammen 1345 g. Zur Durchführung der Bestimmung der AUL 4,8 kPa (0,7 psi) wird das Gewicht des leeren Plexiglas-Zylinders und der Plastikplatte ermittelt und als Wo notiert. Dann werden 0,900 ± 0,005 g Hydrogel-formendes Polymerisat (Korngrößenverteilung 106 - 850 μm) in den Plexiglas-Zylinder eingewogen und möglichst gleichmäßig auf dem Edelstahl-Siebboden verteilt. Anschließend wird die Plastikplatte vorsichtig in den Plexiglas-Zylinder hineingelegt und die gesamte Einheit gewogen; das Gewicht wird als $W_a$ notiert. Nun wird das Gewicht auf die Plastikplatte in dem Plexiglas-Zylinder gestellt. In die Mitte der Petrischale mit einem Durchmesser von 200 mm und einer Höhe von 30 mm wird eine keramische Filterplatte mit einem Durchmesser von 120 mm und einer Porosität 0 gelegt und soviel 0,9 gew.%-ige Natriumchloridlösung eingefüllt, daß die Flüssigkeitsoberfläche mit der Filterplattenoberfläche abschließt, ohne dass die Oberfläche der Filterplatte bedeckt wird. Anschließend wird ein rundes Filterpapier mit einem Durchmesser von 90 mm und einer Porengröße < 20 μm (S&S 589 Schwarzband von Schleicher & Schüll) auf die keramische Platte gelegt. Der Hydrogel-bildende Polymerisat enthaltende Plexiglas-Zylinder wird mit Plastikplatte und Gewicht nun auf das Filterpapier gestellt und dort für 60 Minuten belassen. Nach dieser Zeit wird die komplette Einheit aus der Petrischale vom Filterpapier herausgenommen und anschließend das Gewicht aus dem Plexiglas-Zylinder entfernt. Der gequollenes Hydrogel enthaltende Plexiglas-Zylinder wird zusammen mit der Plastikplatte ausgewogen und das Gewicht als $W_b$ notiert.

Die Absorption unter Druck (AUL) wird wie folgt berechnet:

$$\text{AUL 4,8 kPa (0,7 psi) [g/g]} = [W_b - W_a] / [W_a - W_0]$$

3. Saline Flow Conductivity (SFC)

Die Testmethode zur Bestimmung der SFC ist in der US 5,599,335 beschrieben.

4. Quellgeschwindigkeit (QG)

Bei dieser Methode wird die anfängliche Quellfähigkeit eines Hydrogels in Abwesenheit von Druckbelastung bestimmt. Zur Bestimmung der Quellgeschwindigkeit werden 3,000 $\pm$ 0,005 g des getrockneten, zu testenden Hydrogels (Feuchtigkeitsgehalt < 5 Gew.-%) in einem Salbentopf (Edelstahl 18/10, mit einem Durchmesser von 200 mm und einer Höhe von 100 mm) vorgelegt und homogen in die Mitte des halbkugelförmigen Bodens verteilt. Anschließend gibt man aus einem Dispenser 60,0 g einer 0,9 gew.%-igen Kochsalzlösung genau in die Mitte zu. Man bestimmt die Zeit t in Sekunden (Stoppuhr mit 1/10-Sekunden Teilung), die zwischen der Zugabe der Kochsalzlösung und der Absorption des letzten Flüssigkeitstropfens durch das Hydrogel vergeht. Üblicherweise gibt man den Mittelwert einer Doppelbestimmung an.

Die Quellgeschwindigkeit (QG) wird wie folgt berechnet:

$$\text{QG [g/g}\cdot\text{s]} = \frac{\text{Menge der absorbierten Salzlösung}}{\text{Menge des trockenen Hydrogels} \cdot \text{Aufsaugzeit}}$$

Die Quellgeschwindigkeit gibt demnach an, wieviel g Salzlösung pro Hydrogel, pro Sekunde absorbiert werden.

II Herstellungsbeispiele

Beispiel 1

In einer Glasflasche mit einem Fassungsvermögen von mind. 2,5 L wurde 1 kg handelsübliches Hydrogel des Typs HySorb® M7910 (BASF AG, vernetztes Natriumpolyacrylat) vorgelegt und mit 0,5 Gew.-% handelsüblichem Hydroxylapatit (vertrieben von der Fa. Budenheim) zunächst grob vorgemischt. Verwendet wurde Hydroxylapatit mit einer durchschnittlichen Korngröße von 3 µm und 5 µm. Danach rollte man die Flasche auf einer Rollbank etwa 30 min. Nach dieser Zeit war die Mischung homogen. Die Testergebnisse der auf diese Weise erhaltenen wasserabsorbierenden Mittel sind in Tabelle 1 angegeben.

Tabelle 1

| Versuch | durchschnittl. Teilchengröße [µm] | CRC [g/g] | AUL 4,8 kPa (0,7 psi) [g/g] | QG [g/ g•s] | SFC x $10^{-7}$ [$cm^3$•s/g] |
|---|---|---|---|---|---|
| A (Vergleich) | - | 28,0 | 23,0 | 0,14 | 41 |
| B + 0,5 % Caphosphat | 5 | 29,0 | 23,6 | 0,20 | 58 |
| C + 0,5 % Caphosphat | 3 | 29,9 | 24,4 | 0,25 | 70 |

Beispiel 2

Das Beispiel 1 wurde wiederholt, jedoch setzte man anstelle des Hydrogels vom Typ HySorb® M7910 die gleiche Menge des handelsüblichen Hydrogels des Typs HySorb® M7900 (vernetztes Natriumpolyacrylat) ein. Die Testergebnisse der so erhaltenen wasserabsorbierenden Mittel sind in Tabelle 2 angegeben.

Tabelle 2

| Versuch | durchschnittl. Teilchengröße [μm] | CRC [g/g] | AUL 4,8 kPa (0,7 psi) [g/g] | QG [g/ g•s] | SFC x $10^{-7}$ [$cm^3$•s/g] |
|---|---|---|---|---|---|
| D (Vergleich) | - | 24,0 | 20,7 | 0,13 | 62 |
| E + 0,5 % Caphosphat | 5 | 24,7 | 21,0 | 0,12 | 133 |

Beispiel 3

Das Beispiel 1 wurde wiederholt, jedoch setzte man unterschiedliche Mengen Calciumtriphosphat mit einer durchschnittlichen Teilchengröße von 5 μm ein. Die Testergebnisse der so erhaltenen wasserabsorbierenden Mittel sind in Tabelle 3 angegeben.

Tabelle 3

| Versuch | CRC [g/g] | AUL 4,8 kPa (0,7 psi) [g/g] | SFC x $10^{-7}$ [$cm^3$•s/g] |
|---|---|---|---|
| F (Vergleich) | 27,9 | 23,1 | 45 |
| G + 0,1 % Ca-phosphat | 30,0 | 24,6 | 54 |
| H + 0,2 % Ca-phosphat | 30,2 | 24,7 | 57 |
| I + 0,4 % Ca-phosphat | 29,0 | 24,1 | 68 |
| J + 0,8 % Ca-phosphat | 29,3 | 24,2 | 78 |

Ersichtlich führt die Oberflächenbehandlung des wasserabsorbierenden Polymerisats mit einem feinteiligen Calciumtriphosphat zu einer Erhöhung der Zentrifugenretensionskapazität, der Absorption unter Druck und der Saline Flow Conductivity.

Beispiel 4

Die folgenden Beispiele veranschaulichen das Aufbringen des wasserunlöslichen Metallphosphats aus einer Suspension. Die Ergebnisse sind in Tabelle 4 angegeben.

Beispiel 4.1 (Vergleich)

1500 g eines handelsüblichen Superabsorbers des Typs ASAP 403 (BASF Corp., Aberdeen) wurden in einem Lödige-Labormischer vorgelegt und mit 75 g Isopropanol mittels einer Zweistoffdüse besprüht. Das erhaltene Polymer wurde danach im Trockenschrank getrocknet.

Beispiel 4.2

1500 g eines handelsüblichen Superabsorbers des Typs ASAP 403 wurden in einem Lödige-Labormischer vorgelegt und mit 4,5 g Tricalciumphosphat (Typ C 13-09, SF, mikrofeines Pulver, Lebensmittelqualität - bezogen von Chemischer Fabrik Budenheim), das in 75 g Isopropanol durch Einrühren suspendiert worden war, mittels einer Zweistoffdüse besprüht. Das erhaltene Polymer wurde danach im Trockenschrank getrocknet.

Beispiel 4.3

1500 g eines handelsüblichen Superabsorbers des Typs ASAP 403 wurden in einem Lödige-Labormischer vorgelegt und mit 4,5 g Tricalciumphosphat (Typ C 13-09, SF), das in 30 g Wasser durch Einrühren suspendiert worden war, mittels einer Zweistoffdüse besprüht. Das erhaltene Pulver wurde danach im Trockenschrank getrocknet.

Beispiel 4.4 (Vergleich)

1500 g eines handelsüblichen Superabsorbers des Typs ASAP 403 wurden in einem Lödige-Labormischer vorgelegt und mit 4,5 g Aerosil des Typs Sipernat 22 S (Fa. Degussa), das in 75 g Isopropanol durch Einrühren suspendiert

worden war, mittels einer Zweistoffdüse besprüht. Das erhaltene Polymer wurde danach im Trockenschrank getrocknet.

Beispiel 4.5 (Vergleich)

1500 g eines handelsüblichen Superabsorbers des Typs ASAP 403 wurden in einem Lödige-Labormischer vorgelegt und mit 4,5 g Aerosil des Typs Sipernat D 17 (Fa. Degussa), das in 75 g Isopropanol durch Einrühren suspendiert worden war, mittels einer Zweistoffdüse besprüht. Das erhaltene Polymer wurde danach im Trockenschrank getrocknet.

Beispiel 4.6 (Vergleich)

1500 g eines handelsüblichen Superabsorbers des Typs ASAP 500 (BASF Corp.) wurden in einem Lödige-Labormischer vorgelegt und mit 60 g Isopropanol mittels einer Zweistoffdüse besprüht. Das erhaltene Polymer wurde nicht getrocknet. Dies ist das Vergleichsmuster zu Beispiel 4.7.

Beispiel 4.7

1500 g eines handelsüblichen Superabsorbers des Typs ASAP 403 wurden in einem Lödige-Labormischer vorgelegt und mit 2,25 g Tricalciumphosphat (Typ C 13-09, SF), das in 60 g Isopropanl durch Einrühren suspendiert worden war, mittels einer Zweistoffdüse besprüht. Das erhaltene Polymer wurde nicht getrocknet.

Tabelle 4

| Beispiel Nr. | CRC [g/g] | AUL 4,8 kPa (0,7 psi) [g/g] | SFC x $10^{-7}$ [$cm^3$•s/g] | QG [g/g•s] |
|---|---|---|---|---|
| 4.1* | 28,7 | 24,3 | 56 | 0,18 |
| 4.2 | 28,8 | 24,2 | 89 | 0,21 |
| 4.3 | 28,1 | 24,0 | 80 | 0,20 |
| 4.4* | 28,7 | 21,5 | 70 | 0,21 |
| 4.5* | 28,6 | 21,6 | 74 | 0,12 |
| 4.6* | 29,2 | 22,6 | 23 | --- |
| 4.7 | 29,5 | 22,9 | 31 | --- |
| * Vergleichsbeispiele | | | | |

Beispiel 5

Die folgenden Beispiele veranschaulichen das Aufbringen des wasserunlöslichen Metallphosphats bei der Oberflächennachvernetzung. Die Ergebnisse sind in Tabelle 5 angegeben (die SFC wurde als Mittelwert von 5 Messungen berechnet).

Herstellbeispiel Hydrogel

In einem 40 1-Plastikeimer wurden 6,9 kg reine Acrylsäure durch Einrühren in verdünnte Natronlauge und unter Kühlen mittels eines Wärmeaustauschers zu 75 mol-% neutralisiert und mit Wasser auf 30 kg Reaktionsmasse verdünnt. Zu dieser Lösung fügte man als Vernetzer 50 g Polyethylenglykol-400-diacrylat unter Rühren hinzu und inertisierte den verschlossenen Eimer durch Durchleiten von Stickstoff. Die Polymerisation wurde dann durch Zugabe von 400 mg Wasserstoffperoxid und 200 mg Ascorbinsäure und 10 g Natriumpersulfat gestartet. Nach Beendigung der Reaktion wurde das Gel mechanisch zerkleinert. Das zerkleinerte Gel wurde dann im Labortrockenschrank bei 150 °C für 3 h getrocknet, mit einem Laborwalzenstuhl gemahlen und schließlich bei 200 bis 850 $\mu$m abgesiebt. Dies war das in den nachfolgenden Beispielen verwendete Grundpolymer.

beispiel 5.1 (Vergleich)

1200 g des Grundpolymers wurden in einem Lödige-Pflugschar-Labormischer mittels einer Zweistoffdüse mit Nachvernetzer-Lösung folgender Zusammensetzung besprüht: 1,5 Gew.-% Isopropanol, 3,5 Gew.-% Wasser, 0,12 Gew.-% 2-Oxazolidinon, jeweils bezogen auf eingesetztes Polymer. Anschließend wurde das feuchte Produkt bei 175

°C für 60 min im Umlufttrockenschrank getempert. Das getrokkente Produkt wurde anschließend bei 850 μm abgesiebt, um Klumpen zu entfernen.

Beispiel 5.2

1200 g des Grundpolymers wurden in einem Lödige-Pflugschar-Labormischer mit Vernetzer-Lösung folgender Zusammensetzung besprüht: 1,5 Gew.-% Isopropanol, 3,5 Gew.-% Wasser, 0,12 Gew.-% 2-Oxazolidinon, 0,2 Gew.-% Tricalciumphosphat (Typ C 13-09, SF), jeweils bezogen auf eingesztes Polymer. Anschließend wurde das feuchte Polymer bei 175 °C für 60 min im Umlufttrockenschrank getempert. Das getrocknete Produkt wurde anschließend bei 850 μm abgesiebt, um Klumpen zu entfernen.

Beispiel 5.3

1200 g des Grundpolymers wurden in einem Lödige-Pflugschar-Labormischer mit Vernetzer-Lösung folgender Zusammensetzung besprüht: 1,5 Gew.-% Isopropanol, 3,5 Gew.-% Wasser, 0,10 Gew.-% Ethylenglykoldiglycidilether, 0,2 Gew.-% Tricalciumphosphat (Typ C 13-09, SF), jeweils bezogen auf eingesztes Polymer. Anschließend wurde das feuchte Polymer bei 150 °C für 60 min im Umlufttrockenschrank getempert. Das getrocknete Produkt wurde anschließend bei 850 μm abgesiebt, um Klumpen zu entfernen.

Beispiel 5.4

1200 g des Grundpolymers wurden in einem Waring-Pflugschar-Labormischer mit 0,30 Gew.-% pulvrigem Tricalciumphosphat (Typ C 13-09, SF) zunächt 30 min gemischt; dann wurde mit Vernetzer-Lösung folgender Zusammensetzung besprüht: 1,5 Gew.-% Isopropanol, 3,5 Gew.-% Wasser, 0,10 Gew.-% Ethylenglykoldiglycidylether, jeweils bezogen auf eingesetztes Polymer. Anschließend wurde das feuchte Produkt bei 150°C für 60 min im Umlufttrockenschrank getempert. Das getrocknete Produkt wurde anschließend bei 850 μm abgesiebt, um Klumpen zu entfernen.

Tabelle 5

| Beispiel Nr. | CRC [g/g] | AUL 4,8 kPa (0,7 psi) [g/g] | SFC x $10^{-7}$ [$cm^3$•s/g] |
|---|---|---|---|
| 5.1* | 31.0 | 24,5 | 18 |
| 5.2 | 30,9 | 24,7 | 32 |
| 5.3 | 31,7 | 24,9 | 34 |
| 5.4 | 31,5 | 25, 0 | 29 |
| * Vergleichsbeispiel | | | |

## Patentansprüche

1. Wasserabsorbierendes Mittel, umfassend Teilchen eines oberflächennachvernetzten wasserabsorbierenden Polymerisats, deren Oberfläche mit einem wasserunlöslichen Metallphosphat einer mittleren Teilchengröße von 2 bis weniger als 400 μm assoziiert ist.

2. Wasserabsorbierendes Mittel nach Anspruch 1, wobei das wasserunlösliche Metallphosphat ein Phosphat der Formel $M_4P_2O_7$, $M_2HPO_4$ oder $M_3PO_4$ umfasst, worin M für ein Äquivalent eines unter Calcium, Magnesium, Strontium, Barium, Zink, Eisen, Aluminium, Titan, Zirkonium, Hafnium, Zinn, Cer, Scandium, Yttrium oder Lanthan oder Gemischen davon ausgewählten Metalls steht.

3. Wasserabsorbierendes Mittel nach Anspruch 2, wobei das wasserunlösliche Metallphosphat unter Calciumhydrogenphosphat, tertiärem Calciumphosphat, Apatit, Rhenaniaphosphat, Thomasmehl, Berlinit oder Gemischen davon ausgewählt ist.

4. Wasserabsorbierendes Mittel nach einem der vorhergehenden Ansprüche, wobei das wasserabsorbierende Polymerisat aufgebaut ist aus

   - 49,9 bis 99,9 Gew.-% wenigstens eines unter monoethylenisch ungesättigten Säuren und deren Salzen ausgewählten Monomers A,
- 0 bis 50 Gew.-% wenigstens eines von dem Monomeren A verschiedenen monoethylenisch ungesättigten Monomers B und
- 0,001 bis 20 Gew.-% wenigstens eines vernetzend wirkenden Monomers C.

**5.** Wasserabsorbierendes Mittel nach einem der vorhergehenden Ansprüche, wobei der Anteil des wasserunlöslichen Metallphosphats 0,001 bis 10 Gew.-%, bezogen auf das Gewicht des wasserabsorbierenden Polymerisats, beträgt.

**6.** Verfahren zur Herstellung eines wasserabsorbierenden Mittels nach einem der vorhergehenden Ansprüche, wobei man

a) ein teilchenförmiges wasserabsorbierendes Polymerisat mit einem feinteiligen wasserunlöslichen Metallphosphat innig vermischt; oder
b) eine Aufschlämmung eines feinteiligen wasserunlöslichen Metallphosphats auf ein teilchenförmiges wasserabsorbierendes Polymerisat aufbringt; oder
c) eine Phosphationen enthaltende erste wässrige Lösung mit einer zweiten wässrigen Lösung in Gegenwart eines teilchenförmigen wasserabsorbierenden Polymerisats in Kontakt bringt, wobei die zweite Lösung ein wasserlösliches Salz eines ein wasserunlösliches Phosphat bildenden Metalls enthält.

**7.** Verfahren nach Anspruch 6, wobei man das teilchenförmige wasserabsorbierende Polymerisat gleichzeitig mit der Maßnahme a), b) oder c) oder danach oberflächennachvernetzt.

**8.** Verfahren nach Anspruch 6 oder 7, wobei das teilchenförmige wasserabsorbierende Polymerisat in Form eines zerkleinerten Hydrogels mit einem Feuchtigkeitsgehalt von 0 Gew.-% bis 90 Gew.-% vorliegt.

**9.** Verwendung des wasserabsorbierenden Mittels nach einem der Ansprüche 1 bis 5 als Absorptionsmittel für Körperflüssigkeiten; zur Herstellung von Hygieneartikeln; oder zur Bodenverbesserung.

## Claims

**1.** A water absorbent comprising particles of a surface post-crosslinked water absorbent polymer whose surface is associated with a water insoluble metal phosphate having a median particle size of from 2 to less than 400 μm.

**2.** The water absorbent according to claim 1, wherein the water insoluble metal phosphate is a phosphate of the formula $M_4P_2O_7$, $M_2HPO_4$ or $M_3PO_4$ wherein M is one equivalent of a metal selected from the group consisting of calcium, magnesium, strontium, barium, zinc, iron, aluminum, titanium, zirconium, hafnium, tin, cerium, scandium, yttrium and lanthanum or mixtures thereof.

**3.** The water absorbent according to claim 2, wherein the water insoluble metal phosphate is selected from the group consisting of calcium hydrogenphosphate, tertiary calcium phosphate, apatite, rhenania phosphate, Thomas flour, berlinite and mixtures thereof.

**4.** The water absorbent according to any preceding claim, wherein said water absorbent polymer is polymerized from:

- from 49.9 to 99.9% by weight of at least one monomer A selected from the group consisting of monoethylenically unsaturated acids and salts thereof,
- from 0 to 50% by weight of at least one monoethylenically unsaturated monomer B other than said monomer A, and
- from 0.001 to 20% by weight of at least one crosslinking monomer C.

**5.** The water absorbent according to any preceding claim, wherein the fraction of said water insoluble metal phosphate is in the range from 0.001 to 10% by weight, based on the weight of said water absorbent polymer.

**6.** A process for producing a water absorbent according to any preceding claim, which comprises

a) intimately mixing a particulate water absorbent polymer with a finely divided water insoluble metal phosphate; or

b) applying a slurry of a finely divided water insoluble metal phosphate to a particulate water absorbent polymer; or
c) contacting a first aqueous solution comprising phosphate ions with a second aqueous solution in the presence of a particulate water absorbent polymer, said second solution comprising a water soluble salt of a metal forming a water insoluble phosphate.

**7.** The process according to claim 6, wherein said particulate water absorbent polymer is surface post-crosslinked concurrently with said measure a), b) or c) or thereafter.

**8.** The process according to claim 6 or 7, wherein said particulate water absorbent polymer is in the form of a comminuted hydrogel having a moisture content of from 0% by weight to 90% by weight.

**9.** The use of the water absorbent of any of claims 1 to 5 as an absorbent for body fluids; for producing hygiene articles; or for soil improvement.

**Revendications**

**1.** Agent absorbant l'eau, comprenant des particules d'un polymère absorbant l'eau post-réticulé en surface, dont la surface est associée à un phosphate métallique insoluble dans l'eau présentant une grosseur moyenne des particules de 2 à moins de 400 μm.

**2.** Agent absorbant l'eau selon la revendication 1, le phosphate métallique insoluble dans l'eau comprenant un phosphate de formule $M_4P_2O_7$, $M_2HPO_4$ ou $M_3PO_4$, M représentant un équivalent d'un métal choisi parmi le calcium, le magnésium, le strontium, le baryum, le zinc, le fer, l'aluminium, le titane, le zirconium, l'hafnium, l'étain, le cérium, le scandium, l'yttrium ou le lanthane ou leurs mélanges.

**3.** Agent absorbant l'eau selon la revendication 2, le phosphate métallique insoluble dans l'eau étant choisi parmi l'hydrogénophosphate de calcium, le phosphate tertiaire de calcium, l'apatite, le phosphate de type "rhénania", la farine Thomas, la berlinite ou leurs mélanges.

**4.** Agent absorbant l'eau selon l'une quelconque des revendications précédentes, le polymère absorbant l'eau étant formé à partir de

- 49,9 à 99,9 % en poids d'au moins un monomère A choisi parmi les acides éthyléniquement monoinsaturés et leurs sels,
- 0 à 50 % en poids d'au moins un monomère B éthyléniquement monoinsaturé, différent du monomère A, et
- 0,001 à 20 % en poids d'au moins un monomère C à effet réticulant.

**5.** Agent absorbant l'eau selon l'une quelconque des revendications précédentes, la proportion de phosphate métallique insoluble dans l'eau étant de 0,001 à 10 % en poids par rapport au poids du polymère absorbant l'eau.

**6.** Procédé pour la réalisation d'un agent absorbant l'eau selon l'une quelconque des revendications précédentes, dans lequel

a) on mélange intimement un polymère absorbant l'eau sous forme de particules avec un phosphate métallique insoluble dans l'eau finement divisé ; ou
b) on applique une suspension d'un phosphate métallique insoluble dans l'eau finement divisé sur un polymère absorbant l'eau sous forme de particules ; ou
c) on met en contact une première solution aqueuse contenant des ions phosphate avec une deuxième solution aqueuse en présence d'un polymère absorbant l'eau sous forme de particules, la deuxième solution contenant un sel soluble dans l'eau d'un métal formant un phosphate insoluble dans l'eau.

**7.** Procédé selon la revendication 6, le polymère absorbant l'eau sous forme de particules étant réticulé en surface simultanément avec la mesure a), b) ou c) ou après celle-ci.

**8.** Procédé selon la revendication 6 ou 7, le polymère absorbant l'eau sous forme de particules se trouvant sous forme d'un hydrogel broyé présentant une teneur en humidité de 0 % en poids à 90 % en poids.

**9.** Utilisation de l'agent absorbant l'eau selon l'une quelconque des revendications 1 à 5 comme absorbant pour des liquides corporels ; pour la préparation d'articles hygiéniques ; ou pour l'amélioration du sol.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 3523617 A
- US 4734478 A
- US 4286082 A
- WO 8700848 A
- WO 9511932 A
- EP 386897 A
- US 5599335 A


### In der Beschreibung aufgeführte Nicht-Patentliteratur


- Modern Superabsorbent Polymer Technology. Wiley-VCH, 1998
- **F.L. BUCHHOLZ ; A.T. GRAHAM.** Modern Superabsorbent Polymer Technology. Wiley-VCH, 1998